Europäisches Patentamt

European Patent Office

Office européen des brevets

(11) Publication number: **0 073 803**
**B1**

# EUROPEAN PATENT SPECIFICATION

(45) Date of publication of patent specification: **10.07.85**

(51) Int. Cl.⁴: **A 61 B 17/32**

(21) Application number: **82900833.3**

(22) Date of filing: **12.03.82**

(86) International application number:
**PCT/NO82/00014**

(87) International publication number:
**WO 82/03168 30.09.82 Gazette 82/23**

(54) **MICROSURGICAL INSTRUMENT.**

(30) Priority: **12.03.81 NO 810849**

(43) Date of publication of application:
**16.03.83 Bulletin 83/11**

(45) Publication of the grant of the patent:
**10.07.85 Bulletin 85/28**

(84) Designated Contracting States:
**BE CH DE FR GB LI LU NL**

(56) References cited:
**US-A-2 521 161**
**US-A-2 944 552**
**US-A-3 815 604**
**US-A-3 882 872**

(73) Proprietor: **SKJAERPE, Finn**
**Nedreveien 1**
**N-4000 Stavanger (NO)**

(72) Inventor: **SKJAERPE, Finn**
**Nedreveien 1**
**N-4000 Stavanger (NO)**

(74) Representative: **Needle, Jacqueline et al**
**PAGE, WHITE & FARRER 5 Plough Place**
**New Fetter Lane**
**London EC4A 1HY (GB)**

Courier Press, Leamington Spa, England.

## Description

The present invention relates to a microsurgical instrument for use in the surgical treatment of glaucoma by means of a novel operational procedure which may be called "selective trabeculectomy".

Glaucoma is an ailment in which the internal pressure in the eyeball is increased. Within the eye aqueous humour is produced at a fairly constant rate. This liquid is drained out through filter-like tissue (trabecular meshwork) in the angle between the iris and the cornea into a collector canal which runs circularly along the transition between the cornea and the sclera (Canal of Schlemm), and from this canal through 20—30 drainage outlets in the eye wall into blood vessels (water veins).

The cause of glaucoma is believed to be a type of "clogging" of the trabecular meshwork, so that the outflow resistance increases. The internal pressure then increases to allow the same volume of liquid to be drained per unit time. All treatment aims at reducing the eye pressure. Such treatment is primarily medical, but when this is intolerable and/or insufficient, surgical treatment is used.

The surgical treatments may be subdivided according to three principles, namely:

1. Operations aimed at the reduced production of aqueous humour.

2. Fistulizing procedures, i.e. surgical provision of artificial slits in the eye walls, through which the liquid may drain out of the eye.

3. Operations on the trabecular meshwork. Existing procedures of this kind are of two types, i.e.:

a) Approach through the anterior chamber of the eye by means of goniotomy or cautery of the trabecular meshwork with laser beams.

b) Approach through the Canal of Schlemm. Such approach consists in opening this canal through a radial incision in the eye wall above the canal and insertion of a blunt probe or probe means with a cutting edge (trabeculotome). This instrument is then manipulated in such a way that it tears open or cuts through the trabecular meshwork into the anterior chamber of the eye. With such procedure a narrow slitlike opening is formed through the trabecular meshwork. Such slits exhibit, however, a considerable tendency to close.

In order to inhibit such closure it is an object of the invention to provide a microsurgical instrument, which through appropriate use may form a permanent opening from the anterior chamber of the eye to the Canal of Schlemm by selective removal of the inner wall of this canal along a certain sector. In this manner the aqueous humour gains direct access to the outer wall of the Canal of Schlemm, which has outlets or drainage canals, so that normal drainage of aqueous humour may be re-established.

Thus, the invention provides a microsurgical instrument for performing selective trabeculectomy in surgical treatment of glaucoma, the instrument including flexible probe means and a cutting member fixed to the same.

US—A—2944552 describes an instrument for treating arteriosclerotic occlusive disease which includes flexible probe means and a cutting member affixed to the same, the cutting member comprising two knife blades protruding in different directions from the probe means and each providing at least one sharp cutting edge.

The present invention is characterised in that each cutting edge is turned towards a free end of the probe means, and in that at least one catching device for cut-away tissue is mounted on the cutting member C behind the sharp cutting edges.

An instrument of the invention may be called a trabeculectome, as it is designed to be pulled through the Canal of Schlemm along a certain peripheral sector of the eye. By this the inner wall of the canal and the corresponding portion of the 0.1 mm thick trabecular meshwork are cut away.

In an embodiment, the probe means is knob-shaped at its extreme end and is made of a flexible material. However, the probe means is still sufficiently rigid to be inserted into and directed through the Canal of Schlemm. The cutting member has at least two cutting edges which are angularly separated to such an extent that the V-form defined fits into the scleral groove in the eye wall, in which the trabecular meshwork is embedded. Advantageously, the cutting member is asymmetrical, the two knife edges extending over different lengths. Thus, one knife blade may project perpendicularly into the anterior chamber towards the iris and this blade is short, whereas the other blade may project obliquely into the anterior chamber and forms a small angle with the back side of the cornea, and this other blade is longer. This feature stabilizes the correct position of the knife.

The probe means of the trabeculectome is directed through an incision in the eye wall into and along the Canal of Schlemm, and is pulled out through another incision at a certain distance from the first one. The cutting member of the trabeculectome is pulled after the probe means through the Canal of Schlemm with the two knife edges projecting into the anterior chamber of the eye and thereby cutting away a strip of the trabecular tissue and the inner wall of the Canal of Schlemm which are located between the cutting edges. This tissue strip is generally removed together with the trabeculectome when it is pulled out through the second incision in the eye wall. Such removal of the tissue strip is secured by the catching device mounted on the cutting member. The catching device may be formed by one or more hooks with the hook opening turned towards a free end of the probe means. In an alternative embodiment, the catching device is formed by a plate member connected to an edgefree rim of the knife blades and preferably formed as a trapping hook, to partly or completely cover the space between the knife blades.

A microsurgical instrument of the invention enables a surgical procedure to be performed which secures a permanent broad opening between the anterior chamber of the eye and the outlets from the Canal of Schlemm by removing the trabecular meshwork and the inner wall of the canal. Tissue removal together with maintained cell casing on the outer wall of the Canal of Schlemm make a closure of this opening improbable, even in the long run.

Experiences with this method to date suggest a low rate of complications, so that the operational indications probably may be extended. This means that the patients may be operated at an earlier stage and thus expensive and troublesome medical treatment is avoided.

Embodiments of the present invention will now be described, by way of example, with reference to the accompanying drawings, in which

Figure 1 shows a first embodiment of an instrument of the invention and shows a cross-section of the cutting member taken at right angles to the longitudinal axis of the probe means,

Figure 2 shows a side elevation of the instrument of Figure 1,

Figure 3 shows a perspective view of the instrument of Figures 1 and 2,

Figure 4 shows a side elevation of a further embodiment of an instrument of the invention, and

Figure 5 shows a cross-section of the cutting member taken at right angles to the longitudinal axis of the probe means of the embodiment of Figure 4.

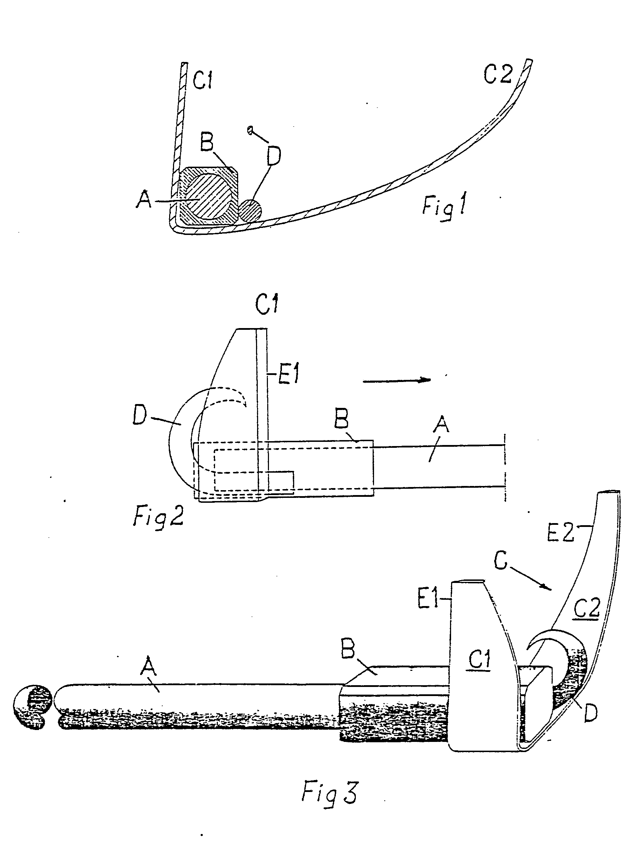

The instrument shown in Figures 1 to 3 comprises a probe A formed by a monofilament of nylon or by a synthetic fibre suture, which is fused at one end to form a rounded knob. The other end of the probe A is inserted into a hole in a metallic holding member B and rigidly clamped to this member. Preferably, the holding member B is made of steel. A cutting member C is formed from a 1/100—5/100 mm thick stainless steel foil, which is finely sharpened at its front edge and bent into an approximate V-form adapted to the local anatomical features of the eye at the Canal of Schlemm and the trabecular meshwork. The cutting member C is fixed to the holding member B and thereby to the probe A by means of a two-component epoxy glue. A point welding technique may also be used if the probe A is mounted on the member B after the welding step.

The cutting member C defines two knife blades C1, C2 which protrude in different directions from the probe A. The sharpened edge defines a cutting edge E1, E2 on each knife blade C1, C2, the cutting edges facing towards said one end of the probe A. An arrow above the probe in Figure 2 shows the pulling direction in use.

Between the knife blades C1, C2, a barbed hook D is fixed to the member B in order to catch the tissue strip which is cut free between the knives. This hook D is also fastened by means of epoxy resin. Finally, a layer of epoxy resin is applied to the joints and transitions between the holding member B and the probe A to provide a completely smooth surface. Teflon and similar polymers may also be used as covering layer and silicone is considered particularly advantageous for this purpose.

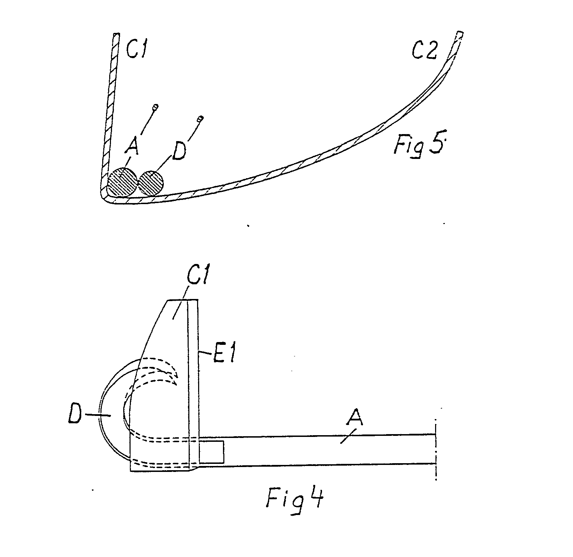

Another embodiment of an instrument of the invention is illustrated in Figures 4 and 5.

In this case the probe A is made of flexible metal and is welded to the cutting member C. The extreme rear end of the probe A is bent forward between the knife blades C1, C2 to form a small sharp hook D. All joints are provided with a cover of epoxy resin and the probe is furnished with a layer of resin at the end to form a finely rounded knob.

The knife blades C1, C2 need not necessarily have a free end. Thus, the extreme ends of the knife blades may well be interconnected to form a closed knife blade ring, e.g. of approximate triangular shape.

The cut-away tissue strip may be captured by closing the rear opening of the knife ring to thereby catch the strip in the "container" thus formed. For example, a plate member (not shown) can be connected to a rim of the blades on which no cutting edge is provided to partly or completely cover the space between the blades. This plate member may be formed as a trapping hook. The instrument must necessarily be quite small to allow the intended surgical treatment of the eye. Thus, in the illustrated embodiments the probe has a diameter of approximately 0.25 mm and a length of the order of magnitude 4—8 cm and rathe freely adaptable to the requirements of the eye surgeon. The shorter knife blade C1 of the cutting member may suitably have a length of about 0.7—1.0 mm, whilst the longer, preferably curved knife blade C2 may have a length of 1.5—1.8 mm. The width of the knife blades may be of the order of magnitude 0.3—0.5 mm.

In the embodiments shown in the drawings and described above the cutting member is fixed at one end of the probe means. However, the cutting member may be fixed to the probe at another location, e.g. on the central part of the same. The portion of the probe projecting from the rear side of the cutting member may then be used for improved steering of the instrument, when it is guided between the two incisions in the eye wall. If the cutting member is located approximately centrally on the probe, the protruding knife blades may further be provided with a cutting edge on both sides, so that the instrument may be effectively pulled in both directions through the Canal of Schlemm.

Although the cutting edges E1, E2 are shown in the drawings to extend substantially at right angles with respect to the longitudinal axis of the probe, these edges may be inclined at an angle between 45° and 90° in the pulling direction with respect to the probe axis. An inwardly directed radial force is then exercised against the tissue strip during the movement of the cutting member through the Canal of Schlemm.

**Claims**

1. Microsurgical instrument for performing selective trabeculectomy in surgical treatment of glaucoma, the instrument including flexible probe means (A) and a cutting member (C) fixed to the probe means and comprising two knife blades (C1, C2) protruding in different directions from the probe means (A) and each providing at least one sharp cutting edge (E1, E2), characterised in that each cutting edge (E1, E2) is turned towards a free end of the probe means (A), and in that at least one catching device (D) for cut-away tissue is mounted on the cutting member (C) behind the sharp cutting edges (E1, E2).

2. An instrument as claimed in Claim 1, characterised in that the two cutting edges (E1, E2) extend for different lengths from the probe means, the shorter edges (E1) being straight and the longer side (E2) having a curvature, preferably adapted to the boundary surface of the tissue to be cut away.

3. An instrument as claimed in Claim 1 or Claim 2, characterised in that the extreme ends of the two knife blades (C1, C2) are interconnected to form a closed knife blade ring.

4. An instrument as claimed in any preceding claim, characterised in that the catching device is formed by one or more hooks (D) with the hook opening turned towards a free end of the probe means.

5. An instrument as claimed in any of Claims 1 to 3, characterised in that the catching device (D) is formed by a plate member connected to an edgefree rim of the knife blades (C1, C2), and preferably formed as a trapping hook, to partly or completely cover the space between the knife blades.

6. An instrument as claimed in any preceding claim, characterised in that the cutting edges (E1, E2) of the knife blades form an angle between 45° and 90° with the longitudinal axis of the probe means (A).

7. An instrument as claimed in any preceding claim, characterised in that the probe means (A) is formed by a synthetic fibre suture with a metallic holder (B) clamped to the suture, e.g. at an extreme end of the same, the cutting member (C) being glued or welded to the holder.

8. An instrument as claimed in any of Claims 1 to 6, characterised in that the probe means (A) is made from flexible metallic material which is welded to the cutting member (C), e.g. at an extreme end, and may be covered with plastics material, preferably silicone.

9. An instrument as claimed in any of Claims 1 to 6, characterised in that the cutting member (C) is moulded in hard plastics material and is integral with or connected to the probe means (A).

**Patentansprüche**

1. Mikrochirurgisches Instrument zum Ausführen der selektiven Trabeculektomie beim chirurgischen Behandeln des Glaukom, mit einem flexiblen Eindringmittel (A) und einem Schneidglied (C), das am Eindringmittel befestigt ist und zwei Messerklingen (C1, C2) umfasst, die in verschiedenen Richtungen von dem Eindringmittel (A) vorstehen und jedes wenigstens eine scharfe Schneidekante (E1, E2) aufweist, dadurch gekennzeichnet, dass jede Schneidekante (E1, E2) gegen ein freies Ende des Eindringmittels (A) hin geformt ist und dass wenigstens eine Greifvorrichtung (D) für weggeschnittens Gewebe auf dem Schneidglied (C) hinter der scharfen Schneidekante (E1, E2) angeordnet ist.

2. Instrument nach Anspruch 1, dadurch gekennzeichnet, dass die zwei Schneidekanten (E1, E2) sich in verschiedenen Längen von dem Eindringmittel wegerstrecken, dass die kürzere Schneidekante gerade ist und dass die längere Schneidekante bogenformig, vorzugsweise an die Grenzfläche des wegzuschneidenden Gewebes angepasst ist.

3. Instrument nach einem der Ansprüche 1 oder 2, dadurch gekennzeichnet, dass die äusseren Enden der zwei Messerklingen (C1, C2) zum Bilden eines geschlossenen Messerklingenringes miteinander verbunden sind.

4. Instrument nach einem der vorangehenden Ansprüche, dadurch gekennzeichnet, dass die Greifvorrichtung durch einen oder mehrere Haken (D) gebildet ist und dass die Hakenöffnung gegen ein freies Endes des Eindringmittels gerichtet ist.

5. Instrument nach einem der Ansprüche 1 bis 3, dadurch gekennzeichnet, dass die Greifvorrichtung (D) durch ein Plattenglied gebildet ist, dass mit dem schneidekantenfreien Rand der Messerklingen (C1, C2) verbunden ist, und vorzugsweise als Fanghaken ausgebildet ist und teilweise oder vollständig den Raum zwischen den Messerklingen einnimmt.

6. Instrument nach einem der vorangehenden Ansprüche, dadurch gekennzeichnet, dass die Schneidekanten (E1, E2) der Messerklingen mit der Längsachse des Eindringmittels (A) einen Winkel von 45° und 90° einschliessen.

7. Instrument nach einem der vorangehenden Ansprüche, dadurch gekennzeichnet, dass das Eindringmittel (A) aus einer Kunstfaserstruktur mit einem metallischen Halter (B) gebildet ist, der an einem Ende der Kunstfaserstruktur angeklemmt ist und dass das Schneidglied (C) an den Halter angeklebt oder angeschweisst ist.

8. Instrument nach einem der Ansprüche 1 bis 6, dadurch gekennzeichnet, dass das Eindringmittel (A) aus einem flexiblen metallischen Material gebildet ist, dessen eines Ende mit dem Schneidglied (C) verschweisst ist und mit Kunststoff, vorzugsweise Silikon, überzogen sein kann.

9. Instrument nach einem der Ansprüche 1 bis 6, dadurch gekennzeichnet, dass das Schneidglied (C) aus hartem Kunststoffmaterial geformt ist und einstückig mit dem Eindringmittel (A) oder mit dem Eindringmittel verbunden ist.

## Revendications

1. Instrument microchirurgical pour réaliser une trabéculectomie sélective dans le traitement chirurgical du glaucome, cet instrument comprenant des moyens de sonde souples (A) et un organe de coupe (C) fixé aux moyens de sonde et comprenant deux lames de couteau (C1, C2) faisant saillie dans des directions différentes par rapport aux moyens de sonde (A) et comportant chacun au moins un tranchant effilé (E1, E2), caractérisé en ce que chaque tranchant (E1, E2) est orienté vers une extrémité libre des moyens de sonde (A) et en ce qu'au moins un dispositif de prise (D) pour le tissu découpé est fixé sur l'organe de coupe (C) derrière les tranchants effilés (E1, E2).

2. Instrument suivant la revendication 1, caractérisé en ce que les deux tranchants (E1, E2) s'étendent suivant des longueurs différentes à partir des moyens de sonde, le bord le plus court (E1) étant droit et le bord le plus long (E2) ayant une courbure, de préférence adaptée à la surface de délimitation du tissu à découper.

3. Instrument suivant l'une ou l'autre des revendications 1 et 2, caractérisé en ce que les extrémités des deux lames de couteau (C1, C2) sont reliées entre elles pour former un anneau à lames de couteau fermé.

4. Instrument suivant l'une quelconque des revendications précédentes, caractérisé en ce que le dispositif de prise est formé par un ou plusieurs crochets (D), l'ouverture du ou des crochets étant orientée vers une extrémité libre des moyens de sonde.

5. Instrument suivant l'une quelconque des revendications 1 à 3, caractérisé en ce que le dispositif de prise (D) est formé par un élément de plaque relié à un bord sans tranchant des lames de couteau (C1, C2), façonné de préférence sous la forme d'un crochet de fixation, de manière à recouvrir partiellement ou totalement l'espace entre les lames de couteau.

6. Instrument suivant l'une quelconque des revendications précédentes, caractérisé en ce que les bords de coupe (E1, E2) des lames de couteau forment un angle de 45° à 90° avec l'axe longitudinal des moyens de sonde (A).

7. Instrument suivant l'une quelconque des revendications précédentes, caractérisé en ce que les moyens de sonde (A) sont formés par une suture en fibres synthétiques, un support métallique (B) étant fixé à la suture, par exemple à une extrémité de celle-ci, l'organe de coupe (C) étant collé ou soudé au support.

8. Instrument suivant l'une quelconque des revendications 1 à 6, caractérisé en ce que les moyens de sonde (A) sont réalisés en une matière métallique souple qui est soudée à l'organe de coupe (C), par exemple à une extrémité, et en ce qu'ils peuvent être recouverts d'une matière plastique, de préférence de la silicone.

9. Instrument suivant l'une quelconque des revendications 1 à 6, caractérisé en ce que l'organe de coupe (C) est moulé dans une matière plastique dure et est solidaire des moyens de sonde (A) ou relié à ces derniers.

Fig 1

Fig 2

Fig 3

1

Fig 5

Fig 4